(19) Europäisches Patentamt
European Patent Office
Office européen des brevets

(11) Publication number: 0 112 071 B1

(12) EUROPEAN PATENT SPECIFICATION

(45) Date of publication of patent specification: 30.12.86

(51) Int. Cl.$^4$: C 07 C 1/20, C 07 C 11/02 // B01J29/28

(21) Application number: 83307094.9

(22) Date of filing: 21.11.83

# (54) Conversion of methanol and methyl ether to light olefins.

(30) Priority: 15.12.82 US 449909
15.12.82 US 449910

(43) Date of publication of application:
27.06.84 Bulletin 84/26

(45) Publication of the grant of the patent:
30.12.86 Bulletin 86/52

(84) Designated Contracting States:
BE DE FR GB IT NL SE

(56) References cited:
EP-A-0 001 695
EP-A-0 040 015
US-A-3 459 676
US-A-3 702 886
US-A-3 709 979
US-A-3 832 449
US-A-4 066 714
US-A-4 172 856

(73) Proprietor: MOBIL OIL CORPORATION
150 East 42nd Street
New York New York 10017 (US)

(72) Inventor: Rosinski, Edward Joseph
Box A
Pedricktown New Jersey 08067 (US)
Inventor: Rubin, Mae Koenig
50 Belmont Avenue
Bala Cynwyd Pennsylvania 19004 (US)
Inventor: May-Som Wu, Margaret
7 Warrenton Way
Belle Mead New Jersey 08802 (US)

(74) Representative: West, Alan Harry
Mobil Court 3 Clements Inn
London WC2A 2EB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 112 071 B1

**Description**

This invention relates to an improved process for converting methanol and/or methyl ether to light olefins over particular types of crystalline aluminosilicate zeolite catalysts.

A remarkable growth in the production of synthetic fibers, plastics and rubber has taken place in recent decades. Such growth, to a large extent, has been supported and encouraged by an expanding supply of inexpensive petroleum raw materials such as ethylene and propylene. However, increasing demand for these light olefins has, from time to time, led to periods of shortage, either due to a diminished supply of suitable feedstocks or to limited processing capacity. In any event, it is now considered highly desirable to provide efficient means for converting raw materials other than petroleum to light olefins.

One such non-petroleum source of light olefins is coal-derived methanol and methyl ether. In this respect, it is known that methanol or methyl ether can be catalytically converted to olefin-containing hydrocarbon mixtures by contact under certain conditions with particular types of crystalline zeolite catalyst materials. U.S. Patent 4,025,575, issued May 24, 1977, to Chang et al and U.S. Patent 4,083,889, issued April 11, 1978 to Caesar et al, for example, both disclose processes whereby methanol and/or methyl ether can be converted to an olefin-containing product over a ZSM-5 type (constraint index 1—12) zeolite catalyst. ZSM-5, in fact, converts methanol and/or methyl ether to hydrocarbons containing a relatively high concentration of light ($C_2$ and $C_3$) olefins with prolonged catalyst lifetime before catalyst regeneration becomes necessary.

It is also known that other types of zeolite catalysts can be used to convert methanol and/or methyl ether to olefin-containing hydrocarbon products containing even higher proportions of light olefins than can be realized by methanol/methyl ether conversion over ZSM-5. For example, U.S. Patents 4,079,095 and 4,079,096, both issued March 14, 1978, to Givens, Plank and Rosinski, disclose that zeolites of the erionite-offretite type, and especially ZSM-34, can usefully be employed to promote conversion of methanol and/or methyl ether to products comprising a major amount of $C_2$ and $C_3$ light olefins. However, while erionite-offretite type catalysts are highly selective to light olefins production, such smaller pore zeolites tend to age rapidly in comparison to ZSM-5 when used for methanol/methyl ether conversion.

A further zeolite-catalyst process for the conversion of methanol and/or dimethyl ether to olefins is disclosed in EP—A—40015, in which the zeolite employed is NU-3. Although the X-ray diffraction pattern of NU-3 exhibits similarities with that of ZSM-45 used in the present invention, there are significant differences between the patterns as is evident by comparing Table 1 of EP—A—40015 and Table I of the present specification. Moreover, it is reported in EP—A—40016, which is directed to the composition and synthesis of NU-3, that NU-3 is a relatively small pore zeolite, with a pore size close to 4 · 3A. Thus, NU-3 is found to absorb little or no p-xylene and similarly would not absorb cyclohexane (with a critical diameter of about 6A). By way of contrast, it is apparent from the data given hereinafter that ZSM-45 absorbs significant quantities of cyclohexane and hence would appear to have a different pore structure than NU-3. Moreover, the process disclosed in EP—A—40015 makes no reference to cofeeding of a diluent and, although high yields of lower olefins are obtained, it is clear from examples 2 and 8 that the catalyst ages rapidly.

Thus in spite of the existence of methanol conversion processes utilizing a variety of zeolite catalysts, there is a continuing need to develop new procedures suitable for selectively converting an organic charge comprising methanol and/or methyl ether over zeolites catalysts to light olefin products with both high light olefin selectivity and prolonged catalyst lifetime.

Accordingly, it is an object of the present invention to provide a process for converting methanol and/or methyl ether over a zeolite-based catalyst to olefin-containing products with high selectivity to production of light olefins.

It is a further object of the present invention to provide such a selective process wherein lifetime of the zeolite catalyst is enhanced for methanol/methyl ether conversion.

It is a further object of the present invention to provide such a methanol/methyl ether conversion process employing a particular type of zeolite catalyst, readily available reactants and diluents and commercially practical reaction conditions.

In accordance with the present invention, there is provided a process for converting an organic reactant feed comprising the organic reactants methanol, methyl ether or mixtures thereof to a $C_2$—$C_4$ olefin-containing hydrocarbon reaction product, said process comprising contacting said organic reactant feed in a reaction zone under methanol or methyl ether conversion reaction conditions with a zeolite-based catalyst composition characterized in that said catalyst composition comprises a synthetic porous crystalline zeolite designated ZSM-45, said zeolite having the formula, on an anhydrous basis and in terms of mole ratio of oxides:

$$M_{2/n}O:Al_2O_3:xSiO_2$$

wherein x is greater than 8, wherein M is an organic or metallic cation or is a combination of organic and metallic cations and wherein n is the valence of each respective cation present, said zeolite having a pore structure such as to absorb significant quantities of cyclohexane and said zeolite having an X-ray powder diffraction pattern containing the following significant lines:

| Interplanar D-spacing (A) | Relative intensity, $I/I_o$ |
|---|---|
| 8.02±0.14 | Strong-Very strong |
| 5.07±0.09 | Medium-Strong |
| 4.21±0.08 | Medium-Strong |
| 4.01±0.07 | Strong-Very strong |
| 3.78±0.07 | Medium-Strong |
| 3.11±0.06 | Medium-Strong |
| 2.751±0.05 | Medium-Strong |

and in that the organic reactant feed is contacted with said catalyst in the presence of a gaseous diluent selected from water, hydrogen-containing gas, nitrogen, helium, carbon monoxide, carbon dioxide and mixtures of said diluents.

In a highly preferred embodiment of the invention, the gaseous diluent comprises a hydrogen-containing gas, e.g., hydrogen.

The term "organic reactant feed" as used herein can comprise both the organic material used as reactants, i.e. the organic compounds such as methanol and methyl ether subjected to catalytic conversion to olefins, as well as additional components such as water or other diluents as described more fully hereinafter. Since methanol is miscible with water, the charge to the catalytic reaction zone may actually contain relatively large amounts of water, but only the methanol, methyl ether and associated organic compounds, constitute the reactant portion of the organic reactant feed.

Any methanol product comprising at least 60 wt.% of methanol may be used to provide methanol for the organic reactant feed in this invention. Substantially pure methanol, such as industrial grade anhydrous methanol, is eminently suitable. Crude methanol, which usually contains from 12 to 20 wt.% water, or more dilute solutions, also may be used.

Small amounts of impurities such as higher alcohols, aldehydes, or other oxygenated compounds in the organic reactant feed have little effect on the conversion reaction of this invention. The organic reactant feed may also comprise methyl ether. When this component is present, it can comprise up to 100% of the organic reactant feed or methyl ether can be admixed with methanol and/or water to form the organic reactant feed. For purposes of the present invention, it is contemplated to directly convert methanol and/or methyl ether in the feed to a hydrocarbon mixture characterized by a high content of light olefins, especially ethylene. Such amounts of methyl ether as may be formed concomitantly in the conversion reaction, however, may be recovered and recycled with fresh organic reactant feed.

The organic reactant feed as hereinbefore described is catalytically converted to a light olefin containing hydrocarbon product enriched in ethylene by contact with a zeolite-based catalyst composition comprising a particular type of synthetic porous crystalline zeolite material designated ZSM-45. The lattice of any crystalline zeolite, including the particular ZSM-45 of the present invention, can be described as a rigid three-dimensional framework of $SiO_4$ and $AlO_4$ in which the tetrahedra are cross-linked by the sharing of oxygen atoms whereby the ratio of the total aluminum and silicon atoms to oxygen atoms is 1:2. The electrovalence of the tetrahedra containing aluminum is balanced by a cation, for example an alkali metal or alkaline earth metal cation. The ratio of cationic-valence/aluminum is thus unity. One cation may be exchanged for another in conventional manner. By means of such cation exchange it has been possible to vary the properties of a given zeolite.

Since the cationic content of a zeolite may thus be regarded as ephemeral, the only significant term in its compositional formula is the ratio of lattice silicon to aluminum, usually expressed as silica/alumina ratio. The $SiO_2/Al_2O_3$ ratio of a given zeolite is however often variable. For example, zeolite X can be synthesized with $SiO_2/Al_2O_3$ ratios of from 2 to 3; zeolite Y, from 3 to 6. In some zeolites, the upper limit of the $SiO_2/Al_2O_3$ ratio is unbounded. ZSM-5 is one such example wherein the $SiO_2Al_2O_3$ ratio is at least 5 and up to infinity. U.S. Patent No. 3,941,871 discloses a zeolite made from a reaction mixture containing no added alumina and exhibiting the X-ray diffraction pattern of zeolite ZSM-5. U.S. Patent Nos. 4,061,724, 4,073,865 and 4,104,294 describe zeolites of similar alumina and metal content.

Synthetic zeolites sometimes resemble naturally occurring zeolites. Zeolites ZSM-35 and ZSM-38 are, for instance, ferrierite-type zeolites. Zeolites ZK-20 (U.S. Patent No. 3,459,676) is described as being isostructural with the naturally occurring zeolite levynite. European Patent Application No. EP—A—40,016 describes synthetic zeolite Nu-3 which is of the levynite type. Zeolite ZSM-45 is a high silica form of a levynite family of materials which exhibits a composition and properties which distinguish it from natural levynite. Zeolite ZSM-45 exhibits a characteristic X-ray powder diffraction pattern which distinguishes it from other known synthetic and naturally occurring zeolites. It may be said to be levynite-type, however.

ZSM-45 has the formula, on an anhydrous basis, in terms of mole ratios of oxides:

$$M_{2/n}O:Al_2O_3:xSiO_2$$

in which x is 8 or greater and M is an organic or metallic cation of valence n, which cation serves to balance the electrovalence of the tetrahedral aluminum atoms. Zeolite ZSM-45 has an X-ray diffraction pattern containing the following significant lines:

| Interplanar D-spacing (Å) | Relative intensity, $I/I_o$ |
|---|---|
| 8.02±0.14 | Strong-Very strong |
| 5.07±0.09 | Medium-Strong |
| 4.21±0.08 | Medium-Strong |
| 4.01±0.07 | Strong-Very strong |
| 3.78±0.07 | Medium-Strong |
| 3.11±0.06 | Medium-Strong |
| 2.751±0.05 | Medium-Strong |

The derivation of such lines in the X-ray pattern is described more fully hereinafter.

In the as-synthesised form, the zeolite ZSM-45 usually conforms to the formula, on an anhydrous basis and in terms of moles ratios of oxides:

$$(0.8\text{—}1.8)R_2O:(0.0\text{—}0.3)Na_2O:(0.0\text{—}0.5)K_2O:Al_2O_3:xSiO_2$$

wherein R is a monovalent organic cation derived from a 2-(hydroxyalkyl) trialkylammonium compound where alkyl is composed of one or two carbon atoms and x is greater than 8, usually from 8 to 100. In such an empirical formula for the as-synthesized zeolite, it is understood that there must always be sufficient R, Na and/or K cations to completely balance the electrovalence of the lattice aluminum. In those instances wherein greater amounts of R, Na and/or K are present than are necessary to balance the aluminum charge, the excess amount of R, Na and/or K is present in the zeolite in the form of occluded compounds formed from these cations.

The as-synthesized form of ZSM-45 has the X-ray diffraction pattern of Table 1 hereinbelow.

TABLE 1

| Interplanar D-spacing (Å) | Relative intensity, $I/I_o$ |
|---|---|
| 10.16±0.18 | Weak |
| 8.02±0.14 | Strong-Very strong |
| 7.56±0.14 | Weak |
| 6.55±0.12 | Medium-Very strong |
| 5.66±0.10 | Weak |
| 5.50±0.10 | Weak |
| 5.07±0.09 | Medium-Strong |
| 4.95±0.09 | Weak |
| 4.21±0.08 | Medium-Strong |
| 4.01±0.07 | Strong-Very strong |
| 3.78±0.07 | Medium-Strong |
| 3.60±0.06 | Weak |

TABLE 1 (contd.)

| Interplanar D-spacing (Å) | Relative intensity, $I/I_o$ |
|---|---|
| 3.54±0.06 | Weak-Medium |
| 3.42±0.06 | Weak |
| 3.27±0.06 | Medium |
| 3.11±0.06 | Medium-Strong |
| 3.03±0.05 | Weak |
| 2.812±0.05 | Weak |
| 2.751±0.05 | Medium-Strong |
| 2.583±0.05 | Weak |
| 2.535±0.05 | Weak |
| 2.521±0.05 | Weak |
| 2.475±0.04 | Weak |
| 2.405±0.04 | Weak |
| 2.362±0.04 | Weak |
| 2.251±0.04 | Weak |
| 2.181±0.04 | Weak |
| 2.133±0.04 | Weak |
| 2.097±0.04 | Weak |
| 2.029±0.04 | Weak |
| 2.006±0.03 | Weak |
| 1.889±0.03 | Weak |
| 1.859±0.03 | Weak |
| 1.843±0.03 | Weak |
| 1.815±0.03 | Weak |
| 1.765±0.03 | Weak |
| 1.721±0.03 | Weak |
| 1.710±0.03 | Weak |
| 1.650±0.03 | Weak |
| 1.637±0.03 | Weak |
| 1.617±0.03 | Weak |
| 1.606±0.03 | Weak |
| 1.559±0.03 | Weak |

These values were determined by standard techniques. The radiation was the K-alpha doublet of copper and a diffractometer equipped with a scintillation counter and an associated computer was used. The peak heights, I, and the positions as a function of 2 theta, where theta is the Bragg angle, were determined using algorithms on the computer associated with the spectrometer. From these, the relative intensities, 100 $I/I_0$, where $I_0$ is the intensity of the strongest line or peak, and d (obs.) the interplanar spacing in Angstrom units (Å), corresponding to the recorded lines, were determined. In Table 1, the relative intensities are given in terms of the strongest line being taken as 100.0. It should be understood that this X-ray diffraction pattern is characteristic of all the species of Zeolite ZSM-45 compositions. The sodium form as well as other cationic forms reveal substantially the same pattern with some minor shifts in interplanar spacing and variation in relative intensity. Other minor variations can occur, depending on the silicon to aluminum ratio of the particular sample, as well as its degree of thermal and/or steam treatment.

The Zeolite ZSM-45 used herein sorbs significant amounts of commonly used test adsorbate materials, i.e. cyclohexane, n-hexane and water, whereas naturally occurring levynite is not expected to adsorb cyclohexane due to its pore structure. Sorption capacities for Zeolite ZSM-45 will range at room temperature as follows:

| Adsorbate | Capacity, wt. percent |
|---|---|
| Cyclohexane | 2.2—7.0 |
| n-Hexane | 7.3—12.9 |
| Water | 14.5—22.9 |

The original organic and alkali metal cations of the as-synthesized ZSM-45 can be replaced at least in part in accordance with techniques well known in the art, with other cations. Such techniques include thermal treatment and ion exchange. Preferred replacing cations include metal ions, hydrogen ions, hydrogen precursor, e.g. ammonium, ions and mixtures thereof. Particularly preferred cations are those which render the ZSM-45 catalytically active for methanol conversion. These include hydrogen, ammonium, rare earth metal and metals of Groups IA, IIA, IIIA, IVA, IB, IIB, IIIB, IVB and VIII of the Periodic Chart of the Elements. The Zeolite ZSM-45 is preferably employed in the methanol conversion process herein in either the ammonium or hydrogen form. The ZSM-45 zeolite may also be utilized in a form wherein some of the active sites have been exchanged with alkali metal, alkaline earth metal or other metal cations. However, the zeolite must contain some acid sites to be effectively utilized in the process of the present invention.

Thermal treatment of the as-synthesized zeolite is generally employed to remove all or part of the organic constituent. Such thermal treatment can for example be conducted at temperatures in excess of 250°C for a period of time sufficient to remove the organic constituent to the desired extent. Following thermal treatment the zeolite can then be subjected to ion exchange. A typical ion exchange technique would be to contact the synthetic ZSM-45 with a salt of the desired replacing cation or cations. Examples of such salts include the halides, e.g. chlorides, nitrates and sulfates.

The Zeolite ZSM-45 useful in the methanol/methyl ether conversion process herein can advantageously be dehydrated, at least partially, prior to use. This can be done by heating the zeolite to a temperature in the range of 200°C to 595°C in an inert atmosphere, such as air, nitrogen, etc. and at atmospheric, subatmospheric or superatmospheric pressures for between 30 minutes and 48 hours. Dehydration can also be performed at room temperature merely by placing ZSM-45 in a vacuum, but a longer time is required to obtain a sufficient amount of dehydration.

The Zeolite ZSM-45 for use in the present process can be prepared from a reaction mixture containing sources of alkali metal ions (K and Na), an oxide of aluminum, an oxide of silicon, an organic cation (R) derived from a 2-(hydroxyalkyl)trialkylammonium compound wherein alkyl is composed of one or two carbon atoms and water and having a composition, in terms of mole ratios of oxides and cations, falling within the following ranges:

| Reactants | Useful | Preferred |
|---|---|---|
| $SiO_2/Al_2O_3$ | 10—150 | 15—80 |
| $OH^-/SiO_2$ | 0.3—1.0 | 0.3—0.8 |
| $H_2O/OH^-$ | 20—100 | 20—80 |
| R/R+(K+Na) | 0.1—0.8 | 0.2—0.7 |
| K/K+Na | 0.0—0.8 | 0.05—0.3 |

wherein R is as above defined.

Crystallization of the Zeolite ZSM-45 can be carried out at either static or stirred condition in a suitable reactor vessel, such as for example, polypropylene jars or teflon lined or stainless steel autoclaves. A useful range of temperatures for crystallization is from about 80°C to about 350°C for a time of about 12 hours to about 200 days. Thereafter, the crystals can be separated from the liquid and recovered. The composition can be prepared utilizing materials which supply the appropriate oxides. Such compositions may include sodium silicate, silica hydrosol, silica gel, silicic acid, sodium hydroxide, a source of aluminum, and an appropriate organic compound. It should be realized that the reaction mixture component oxides can be supplied from more than one source. The reaction mixture can be prepared either batchwise or continuously. Crystal size and crystallization time of the crystalline Zeolite ZSM-45 will vary with the nature of the reaction mixture employed and the crystallization conditions.

In all cases, synthesis of the ZSM-45 crystals is facilitated by the presence of at least 0.01%, preferably 0.10% and still more preferably 1%, seed crystals (based on total weight) of crystalline product.

The 2-(hydroxyalkyl)trialkylammonium compound may be the hydroxide or halide, e.g. chloride, iodide or bromide. When the compound is 2-(hydroxyethyl)trimethylammonium chloride, it is called choline chloride, a preferred source of organic cations (R) in the synthesis of Zeolite ZSM-45.

The ZSM-45 crystals so prepared can be shaped into a wide variety of particle sizes. Generally speaking, the particles can be in the form of a powder, a granule, or a molded product, such as an extrudate having particle size sufficient to pass through a 2 mesh (Tyler) screen and be retained on a 400 mesh (Tyler) screen. In cases where the catalyst is molded, such as by extrusion, the crystals can be extruded before drying or partially dried and then extruded.

It may be desirable to incorporate the Zeolite ZSM-45 with another material resistant to the temperatures and other conditions employed in the methanol/methyl ether conversion process embodiments of the present invention. Such materials include active and inactive material and synthetic or naturally occurring zeolites as well as inorganic materials such as clays, silica and/or metal oxides, e.g. alumina. The latter may be either naturally occurring or in the form of gelatinous precipitates or gels including mixtures of silica and metal oxides. Use of a material which is active in conjunction with the ZSM-45 crystal, i.e. combined therewith, may improve the conversion and/or selectivity of the catalyst in certain methanol conversion process embodiments. Inactive materials suitably serve as diluents to control the amount of conversion in a given process so that products can be obtained economically and orderly without employing other means for controlling the rate of reaction. These materials may be incorporated into naturally occurring clays, e.g. bentonite and kaolin, to improve the crush strength of the catalyst under commercial operating conditions. Said materials, i.e. clays, oxides, etc., function as binders for the catalyst. It is desirable to provide a catalyst composite having good crush strength because in commercial use it is desirable to prevent the catalyst from breaking down into powder-like materials. These clay binders have been employed normally only for the purpose of improving the crush strength of the catalyst.

Naturally occurring clays which can be composited with the ZSM-45 crystalline material include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with the ZSM-45 crystal also include inorganic oxides, notably alumina.

In addition to the foregoing materials, the zeolite ZSM-45 can be composited with a porous matrix material such as silica-alumina, silica-magnesia, silica-zirconia, silica-thoria, silica-beryllia, silica-titania as well as ternary compositions such as silica-alumina-thoria, silica-alumina-zirconia silica-alumina-magnesia and silica-magnesia-zirconia. The relative proportions of finely divided crystalline material and inorganic oxide gel matrix vary widely, with the zeolite content ranging from 1 to 90% by weight and more usually, particularly when the composite is prepared in the form of beads, in the range of 2 to 80 weight % of the composite.

As noted, the ZSM-45 zeolite containing catalyst compositions as hereinbefore described are especially useful for the selective conversion of methanol and/or methyl ether to a light olefin ($C_2$—$C_4$)-containing hydrocarbon product particularly enriched in ethylene. By utilizing a selected combination of the particular ZSM-45 based catalyst along with particular methanol/methyl ether conversion reaction conditions including the presence of certain gaseous diluents, methanol/methyl ether conversion processes can be realized which are more selective to light olefin production than are similar processes employing other types of zeolite catalysts. Processes utilizing these particular ZSM-45 based catalysts and certain reaction conditions are also more resistant to catalyst aging or provide enhanced conversion of methanol and/or methyl ether-containing feed to olefins in comparison with other known zeolite-catalyzed methanol/methyl ether conversion processes. The process of the present invention can, in fact, yield a light olefin-containing product wherein ethylene is the major light olefin produced.

In accordance with the present process invention, a chargestock comprising methanol (methyl alcohol), methyl ether, methanol/methyl ether mixtures can be contacted in the vapor phase with the ZSM-45-based catalyst materials hereinbefore described in a reaction zone in the presence of a gaseous diluent and under reaction conditions suitable for effecting conversion of methanol and/or methyl ether to olefins. Such conditions include an operating temperature between 260°C (~500°F) and 540°C (~1000°F), preferably 300°C and 450°C; a pressure between $10^4$ Pa (0.1 atmosphere) and $3.0\times10^6$ Pa (300 atmospheres)

preferably $5.0\times10^5$ Pa and $10^6$ Pa; and a weight hourly space velocity (WHSV) of the organic reactants between 0.1 and 30, preferably 1 and 10.

The gaseous diluent is co-fed into the reaction zone along with the organic oxygenate reactant. Such diluents include, for example, hydrogen-containing gas, nitrogen, helium, water, carbon monoxide, carbon dioxide, or mixtures of these gases. Preferred diluents are water and/or hydrogen. The gaseous diluent is conveniently co-fed to the reaction zone using a weight hourly space velocity (WHSV) of from 0.003 to 20, preferably from 0.01 to 10. Generally the molar ratio of gaseous diluent to the organic reactants ranges from 0.05:1 to 40:1, more preferably from 0.1:1 to 20:1.

Water (steam) is an especially useful diluent. Selectivity of the methanol conversion reaction for production of light ($C_2$—$C_4$) olefins can be improved for example by contacting the feedstock with the hereinbefore described zeolite based catalyst in the presence of up to 20 moles, and preferably from 1 to 10 moles of steam per mole of organic reactant. Such steam contact is made in the reaction zone under the methanol/methyl ether conversion conditions hereinbefore described. Such steam may be provided directly by injecting the requisite amount of water or steam into the reaction zone. Alternatively, steam may be provided totally or in part by water present in the feedstock in a molar ratio of water to organic reactants of up to 20:1, preferably from 1:1 to 10:1. Such water in the feedstock to the reaction zone, of course, forms steam in the reaction zone under the conversion conditions of the present invention. Steam can thus be passed to the reaction zone with a WHSV of from 1 to 20, more preferably of from 2 to 10.

Another especially preferred gaseous diluent is a hydrogen-containing gas which provides a reducing atmosphere in the methanol conversion reaction zone. Such a hydrogen-containing gas can be selected from substantially pure hydrogen and mixtures of hydrogen and carbon monoxide such as are found in synthesis gas. Whatever the nature of particular hydrogen-containing gaseous diluent utilized, it has been found that hydrogen-containing gaseous diluents of the type described herein can usefully be employed to prolong the lifetime and selectivity of the ZSM-45 zeolite based methanol/methyl ether conversion catalyst when such catalysts and diluents are employed under reaction conditions suitable for the selective conversion of methanol/methyl ether to light olefins.

Conversion reaction conditions, as well as the nature of the particular catalyst composition and diluent employed, can affect the selectivity of the present methanol/methyl ether conversion process to light olefin and ethylene production as well as percent conversion of organic reactants to hydrocarbon product and can also affect catalyst aging characteristics. However, it has been discovered that for a given set of reaction conditions the ZSM-45 based catalyst used in the present invention, and especially a ZSM-45 based catalyst used in combination with a hydrogen-containing gaseous diluent, will generally provide improved methanol conversion, light olefin selectivity and/or catalyst aging performance in comparison with similar prior art processes employing either other zeolites or diluent-free reaction conditions.

It should be noted that "catalyst lifetime" as used herein refers to the length of time on stream during which a catalyst can be employed before catalytic activity drops to a level such that catalyst regeneration becomes commercially desirable or necessary. A convenient measurement of catalyst lifetime would be the length of time a catalyst can be employed in the process of the present invention before methanol/methyl ether conversion in the process drops from its initial value to an arbitrarily set lower conversion value, e.g., 50% conversion. For purposes of the present invention, enhanced catalyst lifetime refers to the extension of the catalyst lifetime which can be realized with a process using the ZSM-45 gaseous diluent combination in comparison with prior art methanol/methyl ether conversion processes wherein this particular zeolite/diluent combination is not employed.

The methanol and/or methyl ether conversion process described herein may be carried out as a batch-type, semi-continuous or continuous operation utilizing a fixed, fluidized or moving bed catalyst system. A preferred embodiment entails use of a catalyst zone wherein the alcohol or ether charge optionally together with added water is passed concurrently or countercurrently through a fluidized or moving bed of particle-form catalyst. The latter after use may be conducted to a regeneration zone wherein catalyst can be regenerated by any of the conventional regeneration methods known to the art and thereafter recycled to the conversion zone for further contact with the methanol and/or ether containing feed.

The product stream in the process of the invention contains steam and a hydrocarbon mixture of paraffins and olefins, which mixture can be substantially devoid of aromatics. As noted, the mixture is particularly rich in light olefins, and especially rich in ethylene. Thus, the predominant hydrocarbon product constitutes valuable petrochemicals. The steam and hydrocarbon products may be separated from one another by methods well known in the art. In a preferred embodiment of the invention, the unconverted methanol and/or methyl ether, as well as at least part of the water in the product, can be recycled to the reaction zone.

In order to more fully illustrate the nature of the invention and the manner of practicing same, the following examples are presented. In the examples, whenever adsorption data are set forth for comparison of sorptive capacities of ZSM-45 for water, cyclohexane and/or n-hexane, they were determined as follows:

A weighed sample of the calcined ZSM-45 adsorbant was contacted with the desired pure adsorbate vapor in an adsorption chamber, evacuated to less than 133.22 Pa (1 mm Hg) and contacted with 1600 Pa (12 mm Hg) of water vapor or 2666.55 Pa (20 mm Hg) of n-hexane, or cyclohexane vapor, pressures less than the vapor-liquid equilibrium pressure of the respective adsorbate at room temperature. The pressure

was kept constant (within about ±66.66 Pa) by addition of adsorbate vapor controlled by a manostat during the adsorption period, which did not exceed about 8 hours. As adsorbate was adsorbed by the zeolite crystal, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant.

When Alpha Value for the ZSM-45 zeolite is determined, it is noted that the Alpha Value is an approximate indication of the catalytic activity, e.g., cracking activity, of the catalyst compared to a standard catalyst and it gives the relative rate constant (rate of normal hexane conversion per volume of catalyst per unit time). It is based on the activity of the highly active silica-alumina cracking catalyst taken as an Alpha Value of 1 (Rate Constant=0.016 $sec^{-1}$). The Alpha Test is described in U.S. Patent 3,354,078 and in The Journal of Catalysis, Vol. IV, pp. 522—529 (August 1965).

Example 1

Three separate components were prepared to comprise ingredients as follows:

A. 15.45 grams of $Al_2(SO_4)_3 \cdot 18H_2O$
   3.48 grams of $H_2SO_4$
   90.2 grams of $H_2O$
B. 135.4 grams of Q-Brand sodium silicate (28.5 wt. percent $SiO_2$, 8.8 wt. percent $Na_2O$ and 62.7 wt. percent $H_2O$)
   2.0 grams of 86.4 wt. percent KOH
   11.0 grams of $H_2O$
C. 38.0 grams of choline chloride

Component C was added to component B and A was then added to the whole. The whole composition was then mixed and the mixture was transferred to a polypropylene jar. Crystallization occurred under static conditions at 99°C over 197 days. The crystalline product was separated, washed and dried and identified by X-ray diffraction analysis to be about 90 percent zeolite ZSM-45 plus about 10 percent unidentified impurities. The complete X-ray pattern data for this zeolite is presented in Table 2, hereinafter.

Chemical analysis of the zeolite product of this example proved it to have the following composition:

| Component | Wt. percent |
|---|---|
| N | 2.07 |
| Na | 0.28 |
| K | 0.35 |
| $Al_2O_3$ | 7.40 |
| $SiO_2$ | 89.90 |
| Ash | 80.50 |
| $SiO_2/Al_2O_3$, molar | 20.7 |

Sorption capacities of the zeolite product of this example, calcined at 538°C, were:

| Adsorbate | Capacity, wt. percent |
|---|---|
| Cyclohexane, 20 Torr (2666.44 Pa) | 3.5 |
| n-Hexane, 20 Torr (2666.44 Pa) | 12.9 |
| Water, 12 Torr (1600 Pa) | 18.3 |

The surface area of the zeolite ZSM-45 product of this example was measured to be 514 $m^2$/gram.

TABLE 2

| Degrees two theta | Interplanar d-spacing (Å) | Relative intensity, $I/I_o$ |
|---|---|---|
| 7.72 | 11.45 | 6 |
| 8.60 | 10.28 | 13 |
| 10.91 | 8.11 | 50 |
| 11.45 | 7.73 | 3 |
| 11.65 | 7.60 | 6 |
| 13.41 | 6.60 | 36 |
| 15.60 | 5.68 | 6 |
| 16.00 | 5.54 | 9 |
| 17.38 | 5.10 | 81 |
| 17.84 | 4.97 | 12 |
| 20.98 | 4.23 | 51 |
| 22.08 | 4.03 | 100 |
| 23.44 | 3.80 | 45 |
| 25.05 | 3.55 | 13 |
| 25.53 | 3.49 | 5 |
| 25.94 | 3.43 | 5 |
| 27.12 | 3.29 | 20 |
| 28.53 | 3.13 | 47 |
| 29.38 | 3.04 | 6 |
| 31.70 | 2.823 | 8 |
| 32.45 | 2.759 | 40 |
| 34.57 | 2.595 | 10 |
| 35.25 | 2.546 | 2 |
| 35.46 | 2.531 | 2 |
| 38.08 | 2.363 | 2 |
| 38.41 | 2.344 | 1 |
| 39.80 | 2.265 | 3 |
| 40.50 | 2.227 | 1 |
| 41.25 | 2.189 | 3 |
| 42.23 | 2.140 | 3 |
| 43.00 | 2.103 | 8 |

TABLE 2 (contd.)

| Degrees two theta | Interplanar d-spacing (Å) | Relative intensity, $I/I_o$ |
|---|---|---|
| 44.52 | 2.035 | 2 |
| 45.10 | 2.010 | 4 |
| 46.89 | 1.938 | 1 |
| 47.32 | 1.921 | 1 |
| 48.02 | 1.895 | 6 |
| 48.90 | 1.863 | 6 |
| 49.40 | 1.845 | 3 |
| 50.10 | 1.821 | 7 |
| 51.61 | 1.771 | 12 |
| 52.42 | 1.745 | 1 |
| 53.00 | 1.728 | 2 |
| 53.50 | 1.713 | 1 |
| 54.10 | 1.695 | 1 |
| 55.00 | 1.670 | 1 |
| 55.58 | 1.653 | 3 |
| 56.02 | 1.642 | 8 |
| 56.82 | 1.620 | 3 |
| 57.35 | 1.607 | 2 |
| 59.15 | 1.562 | 5 |

Example 2

About 10 grams of the zeolite ZSM-45 product of Example 1 was calcined in air at 538°C for 10 hours and then contacted with 10 cc/gram zeolite of 5 percent ammonium chloride solution at 85°C five separate times. The resulting zeolite was then dried at about 110°C and calcined at 538°C for 10 hours in air. It was then submitted for evaluation in the Alpha Test, described hereinbefore. Its Alpha Value proved to be 14.

Example 3

Three separate components were prepared to comprise ingredients as follows:

A. 15.45 grams of $Al_2(SO_4)_3 \cdot 18H_2O$
   3.48 grams of $H_2SO_4$
   90.2 grams of $H_2O$

B. 135.4 grams of Q-Brand sodium silicate (28.5 wt. percent $SiO_2$, 8.8 wt. percent $Na_2O$ and 62.7 wt. percent $H_2O$)
   2.0 grams of 86.4 wt. percent KOH
   50.0 grams of $H_2O$

C. 38.0 grams of choline chloride

Component C was added to component B and component A was then added to the whole. The whole composition was then mixed and the mixture was transferred to a polypropylene jar. Crystallization occurred under static conditions at 99°C over 152 days. The crystalline product was separated, washed and dried and identified by X-ray diffraction analysis to be zeolite ZSM-45.

Chemical analysis of the zeolite product of this example proved it to have the following composition:

| Component | Wt. percent |
|---|---|
| N | 2.25 |
| Na | 0.43 |
| K | 0.025 |
| $Al_2O_3$ | 7.60 |
| $SiO_2$ | 89.00 |
| Ash | 78.90 |

Sorption capacities of the zeolite product of this example, calcined at 538°C, were:

| Adsorbate | Capacity, wt. percent |
|---|---|
| Cyclohexane, 20 Torr (2666.44 Pa) | 3.8 |
| n-Hexane, 20 Torr (2666.44 Pa) | 11.3 |
| Water, 12 Torr (1600 Pa) | 16.2 |

The surface area of the zeolite product of this example was measured to be 467 $m^2$/gram.

Example 4

About 10 grams of the zeolite ZSM-45 product of Example 3 was calcined in air at 538°C for 10 hours and then contacted with 5 percent ammonium chloride solution, dried and calcined as in Example 2. It was then submitted for evaluation in the Alpha Test. Its Alpha Value proved to be 6.

Example 5

Three separate components were prepared to comprise ingredients as follows:

A. 28.7 grams of sodium aluminate
   36.0 grams of NaOH
   10.0 grams of 85 wt. percent KOH
   450.0 grams of $H_2O$
B. 650.0 grams of colloidal silica (30 wt. percent)
C. 190.0 grams of choline chloride

Component C was added to component A and component B was then added to the whole. The whole composition was then mixed and the mixture was transferred to a polypropylene jar. Crystallization occurred under static conditions at 121°C over 21 days. The crystalline product was separated, washed and dried and identified by X-ray diffraction analysis to be about 85 percent zeolite ZSM-45 plus about 15 percent unidentified impurities. The complete X-ray pattern data for this zeolite is presented in Table 3, hereinafter.

TABLE 3

| Degrees two theta | Interplanar D-spacing (Å) | Relative intensity, $I/I_o$ |
|---|---|---|
| 7.78 | 11.36 | 14 |
| 8.67 | 10.20 | 11 |
| 11.00 | 8.04 | 37 |
| 11.69 | 7.57 | 8 |
| 12.60 | 7.03 | 4 |
| 13.15 | 6.73 | 8 |
| 13.49 | 6.60 | 37 |
| 15.55 | 5.70 | 6 |
| 16.05 | 5.52 | 9 |
| 17.45 | 5.08 | 65 |
| 17.88 | 4.96 | 20 |
| 19.45 | 4.56 | 6 |
| 20.72 | 4.29 | 20 |
| 21.07 | 4.22 | 47 |
| 22.12 | 4.02 | 100 |
| 23.49 | 3.79 | 62 |
| 25.14 | 3.54 | 38 |
| 25.91 | 3.44 | 18 |
| 26.90 | 3.31 | 10 |
| 27.19 | 3.28 | 27 |
| 28.35 | 3.15 | 13 |
| 28.63 | 3.12 | 40 |
| 29.40 | 3.04 | 7 |
| 31.54 | 2.84 | 11 |
| 31.75 | 2.819 | 10 |
| 32.51 | 2.754 | 35 |
| 34.65 | 2.589 | 6 |
| 35.50 | 2.529 | 2 |
| 36.25 | 2.478 | 5 |
| 38.10 | 2.362 | 3 |
| 39.80 | 2.265 | 2 |

TABLE 3 (contd.)

| Degrees two theta | Interplanar D-spacing (Å) | Relative intensity, $I/I_o$ |
|---|---|---|
| 41.25 | 2.189 | 2 |
| 42.28 | 2.138 | 3 |
| 43.05 | 2.101 | 7 |
| 45.17 | 2.007 | 3 |
| 48.11 | 1.891 | 5 |
| 48.90 | 1.863 | 4 |
| 49.45 | 1.843 | 3 |
| 50.19 | 1.817 | 6 |
| 51.74 | 1.767 | 11 |
| 52.94 | 1.730 | 1 |
| 55.68 | 1.651 | 4 |
| 56.15 | 1.638 | 6 |
| 56.94 | 1.617 | 1 |
| 59.26 | 1.559 | 3 |

Chemical analysis of the zeolite product of this example proved it to have the following composition:

| Component | Wt. percent |
|---|---|
| N | 2.34 |
| Na | 0.49 |
| K | 0.58 |
| $Al_2O_3$ | 6.90 |
| $SiO_2$ | 72.60 |
| Ash | 81.50 |

Sorption capacities of the zeolite product of this example, calcined at 538°C, were:

| Adsorbate | Capcity, wt. percent |
|---|---|
| Cyclohexane, 20 Torr (2666.44 Pa) | 5.0 |
| n-Hexane, 20 Torr (2666.44 Pa) | 11.0 |
| Water, 12 Torr (1600 Pa) | 14.5 |

The surface area of the zeolite product of this example was measured to be 484 $m^2$/gram.

Example 6

About 10 grams of the zeolite ZSM-45 product of Example 5 was calcined in air at 538°C for 10 hours and then contacted with 5 percent ammonium chloride solution, dried and calcined as in Example 4. It was then submitted for evaluation in the Alpha Test. Its Alpha Value proved to be 27.

Example 7

Three separate components were prepared to comprise ingredients as follows:

A. 23.2 grams of $Al_2(SO_4)_3 \cdot 18H_2O$
   5.2 grams of $H_2SO_4$
   135 grams of $H_2O$
B. 203.1 grams of Q-brand sodium silicate (28.5 wt. percent $SiO_2$, 8.8 wt percent $Na_2O$ and 62.7 wt. percent $H_2O$)
   3.0 grams of 86.4 wt. percent KOH
   75.0 grams of $H_2O$
C. 57.0 grams of choline chloride

Component C was added to component B and A was then added to the whole. The whole composition was then mixed and the mixture was transferred to a polypropylene jar. Crystallization occurred under static conditions at 100°C over 151 days. The crystalline product was separated, washed and dried and identified by X-ray diffraction analysis to be 100 percent zeolite ZSM-45. The complete X-ray pattern data for this zeolite is presented in Table 4, hereinafter.

TABLE 4

| Degrees two theta | Interplanar D-spacing (Å) | Relative intensity, $I/I_o$ |
|---|---|---|
| 7.77 | 11.38 | 4 |
| 8.71 | 10.15 | 10 |
| 11.04 | 8.02 | 45 |
| 11.71 | 7.56 | 6 |
| 13.52 | 6.55 | 36 |
| 15.67 | 5.66 | 2 |
| 16.11 | 5.50 | 10 |
| 17.49 | 5.07 | 76 |
| 17.93 | 4.95 | 13 |
| 19.57 | 4.54 | 4 |
| 21.10 | 4.21 | 50 |
| 22.18 | 4.01 | 100 |
| 23.54 | 3.78 | 47 |
| 23.80 | 3.74 | 8 |
| 25.16 | 3.54 | 15 |
| 26.08 | 3.42 | 5 |
| 27.24 | 3.27 | 24 |
| 28.67 | 3.11 | 44 |
| 29.46 | 3.03 | 7 |
| 31.84 | 2.810 | 7 |
| 32.55 | 2.750 | 38 |
| 34.72 | 2.584 | 9 |

TABLE 4 (contd.)

| Degrees two theta | Interplanar D-spacing (Å) | Relative intensity, $I/I_o$ |
|---|---|---|
| 35.42 | 2.534 | 3 |
| 35.62 | 2.520 | 2 |
| 36.28 | 2.476 | 5 |
| 37.42 | 2.403 | 2 |
| 38.22 | 2.355 | 1 |
| 38.48 | 2.339 | 1 |
| 39.95 | 2.257 | 2 |
| 40.50 | 2.227 | 2 |
| 41.36 | 2.183 | 3 |
| 42.40 | 2.132 | 3 |
| 43.12 | 2.098 | 7 |
| 44.70 | 2.027 | 6 |
| 45.22 | 2.005 | 4 |
| 47.35 | 1.920 | 2 |
| 48.16 | 1.889 | 5 |
| 49.03 | 1.858 | 4 |
| 49.50 | 1.841 | 3 |
| 50.23 | 1.816 | 6 |
| 51.76 | 1.766 | 11 |
| 53.01 | 1.727 | 2 |
| 53.75 | 1.705 | 2 |
| 55.68 | 1.651 | 3 |
| 56.15 | 1.638 | 6 |
| 56.99 | 1.616 | 2 |
| 57.39 | 1.605 | 1 |
| 59.30 | 1.558 | 4 |

Chemical analysis of the zeolite product of this example proved it to have the following composition:

| Component | Wt. percent |
|---|---|
| N | 2.26 |
| Na | 0.37 |
| K | 0.79 |
| $Al_2O_3$ | 7.48 |
| $SiO_2$ | 85.8 |
| Ash | 82.8 |
| $SiO_2/Al_2O_3$, molar | 19.5 |

Sorption capacities of the zeolite product of this example, calcined at 538°C, were:

| Adsorbate | Capacity, wt. percent |
|---|---|
| Cyclohexane, 20 Torr (2666.44 Pa) | 3.4 |
| n-Hexane, 20 Torr (2666.44 Pa) | 12.9 |
| Water, 12 Torr (1600 Pa) | 21.1 |

The surface area of the zeolite product of this example was measured to be 471 $m^2$/gram.

Example 8

Two grams of zeolite ZSM-45 product of Example 2 were dried by heating for 1 hour in air flowing at 10 cc/hour at 538°C. The zeolite was mixed with an equal volume of quartz chips (both 14/25 mesh) and the mixture placed in a quartz microreactor equipped with a thermocouple. The reactor was then flushed with air at 10 cc/minute for 105 minutes at 538°C. The air flow was replaced with helium flow at 10 cc/minute while the reactor temperature was reduced to about 371°C. Feedstock composed of 30 wt.% methanol and 70 wt.% water was then passed into the reactor at 3.4 cc/hour for 2 hours at atmospheric pressure. The weight hourly space velocity was 2.8 $hr^{-1}$ and the temperature was maintained at about 371°C. Product collected from the reactor was analyzed for chemical composition. Conversion of methanol was indicated to be 87 wt.%. The product hydrocarbon content comprised 39 wt.% ethylene and 16 wt.% $C_5^+$ components.

Example 9

Two grams of zeolite ZSM-45 prepared as in Example 4 were dried, mixed with quartz chips, placed in the microreactor, air flushed and helium flushed as in Example 8. With the reactor temperature maintained at about 371°C and the pressure at atmospheric, feedstock comprising 30 wt.% methanol and 70 wt.% water was passed into the reactor at a WHSV of 2.4 $hr^{-1}$. The molar ratio of water/methanol in the feedstock was 4/1. Product was collected and analyzed. Conversion of methanol proved to be 46 wt.%. Product hydrocarbon content comprised 44 wt.% ethylene and nil $C_5^+$ components.

Example 10

In this example, two grams of zeolite ZSM-45 prepared as in Example 4 were dried, mixed with quartz chips, placed in the microreactor, air and helium flushed as in Example 9. With the reactor maintained at from about 371°C to about 379°C and the pressure at atmospheric, feedstock of 100% methanol was passed into the reactor at a WHSV of 2.4 $hr^{-1}$. Product was collected and analyzed, showing 79.7 wt.% conversion of methanol to product hydrocarbons comprising:

| Component | wt.% |
|---|---|
| Ethylene | 16.91 |
| Propylene | 39.46 |
| Isobutane | 11.68 |
| Butylene | 22.55 |
| $C_5^+$ hydrocarbons | 9.40 |

Example 11

A ZSM-45 sample, prepared in the general manner described in Examples 1 and 2, is used in its ammonium exchanged form to convert methanol to hydrocarbons in the general manner of Examples 8, 9 and 10. The ZSM-45 used in such conversion has a silica/alumina ratio of about 26 and the following sorption capacity and alkali metal content:

| | |
|---|---|
| Cyclohexane, wt.%: | 3.5 |
| n-Hexane, wt.%: | 11.2 |
| $H_2O$, wt.%: | 18.0 |
| Residual Na, wt.%: | 0.28 |
| Residual K, wt.%: | 0.1 |

In such a procedure, 2 grams of zeolite (no binder) and 4 grams of quartz chips, both of 14/20 mesh size, are mixed and packed into a stainless steel microreactor, equipped with thermocouple. Methanol is fed to the reactor under hydrogen pressure under varying reaction conditions for a period of up to 177 hours. Pressure during the run is maintained at 963 kPa (125 psi). A WHSV for methanol of 0.4 is used. Data points are taken at various times during the run. The total reactor effluent is analyzed, on line, by gas chromatography. Methanol conversion is calculated based on hydrocarbon formation only. Selectivities (wt.%) to hydrocarbon product are calculated on "coke free" basis. A catalyst aging rate defined as the amount of change in percent methanol conversion to hydrocarbon per hour is also computed. Reaction conditions, as well as methanol conversion results for this run, are set forth in Table 5.

TABLE 5
Methanol conversion to light olefins over zeolite ZSM-45

| Run No. | 1 | 2 | 3 | 4 | 5 | 6 | 7 |
|---|---|---|---|---|---|---|---|
| Time on stream, hours | 1—22 | 22—29 | 29—49 | 49—55 | 55—70 | 70—95 | 95—177 |
| Temperature, °C | 350—372 | 391 | 400 | 400 | 402 | 402 | 402 |
| $H_2$/MeOH molar ratio | 10 | 10 | 10 | 4 | 4 | 1.5 | 1 |
| Percent conversion | 22 | 76 | 96 | 95 | 98 | 82 | 61 |
| Product selectivity (wt.%) | | | | | | | |
| $C_2H_4$ | 35.8 | 58.2 | 57.2 | 54.8 | 49.9 | 51.4 | 46.2 |
| $C_3H_6$ | 21.8 | 16.1 | 15.2 | 21.0 | 23.0 | 28.0 | 28.7 |
| $C_2H_4$—$C_4H_8$ | 64.6 | 75.4 | 73.2 | 77.3 | 75.0 | 81.8 | 78.2 |
| $CH_4$—$C_3H_8$ | 29.4 | 23.8 | 26.3 | 22.3 | 24.3 | 17.4 | 20.3 |
| Aging rate, percent per hour | — | 0 | 0 | 0.34 | 0 | 0.63 | 0.24 |

The Table 5 data indicate that for a molar ratio of $H_2$/MeOH of 10 and at 400°C, methanol conversion results of about 95% can be achieved. Selectivities to ethylene and total light olefins are greater than 55% and 75%, respectively, under these conditions. When the molar ratio of $H_2$/MeOH was lowered to 1 (Run No. 7), the catalyst maintains its activity and selectivities for more than 80 hours. During this period, the ethylene selectivity ranges from 40—50%. Total light olefin selectivity is greater than 75%. The aging rate for methanol conversion under this condition is only 0.24% per hour. Such results clearly show the effectiveness of Zeolite ZSM-45 under hydrogen pressure in promoting the selective conversion of methanol to a hydrocarbon product enriched in light olefins.

**Claims**

1. A process for converting an organic reactant feed comprising the organic reactants methanol, methyl ether or mixtures thereof to a $C_2$—$C_4$ olefin-containing hydrocarbon reaction product, said process comprising contacting said organic reactant feed in a reaction zone under methanol or methyl ether conversion reaction conditions with a zeolite-based catalyst composition characterized in that said catalyst composition comprises a synthetic porous crystalline zeolite designated ZSM-45, said zeolite having the formula, on an anhydrous basis and in terms of mole ratio of oxides:

$$M_{2/n}O:Al_2O_3:xSiO_2$$

wherein x is greater than 8, wherein M is an organic or metallic cation or is a combination of organic and metallic cations and wherein n is the valence of each respective cation present, said zeolite having a pore structure such as to absorb significant quantities of cyclohexane and said zeolite having an X-ray powder diffraction pattern containing the following significant lines:

| Interplanar D-spacing (A) | Relative intensity, $I/I_o$ |
|---|---|
| 8.02±0.14 | Strong-very strong |
| 5.07±0.09 | Medium-strong |
| 4.21±0.08 | Medium-strong |
| 4.01±0.07 | Strong-very strong |
| 3.78±0.07 | Medium-strong |
| 3.11±0.06 | Medium-strong |
| 2.751±0.05 | Medium strong |

and in that the organic reactant feed is contacted with said catalyst in the presence of a gaseous diluent selected from water, hydrogen-containing gas, nitrogen, helium, carbon monoxide, carbon dioxide and mixtures of said diluents.

2. A process according to Claim 1 wherein, in the catalyst composition, the ZSM-45 zeolite component, in its as-synthesized form, has a composition on an anhydrous basis and in terms of moles of oxides per mole of alumina, expressed by the formula:

$$(0.8—1.8)R_2O:(0.0—0.3)Na_2O:(0.0—0.5)K_2O:Al_2O_3:xSiO_2$$

wherein R is a monovalent organic cation derived from a 2-(hydroxyalkyl) trialkylammonium compound where alkyl is composed of one or two carbon atoms and x is greater than 8, said ZSM-45 zeolite being further characterized by an X-ray diffraction pattern exhibiting values substantially as set forth in Table 1 of the specification.

3. A process according to Claim 1 or Claim 2 wherein, in the formula for the zeolite, x is from greater than 8 to 100.

4. A process according to Claim 2 or Claim 3 wherein, in the formula for the zeolite, R is an organic cation derived from choline chloride.

5. A process according to any of Claims 2 to 4 wherein said catalyst composition comprises the product of thermal treatment of the as-synthesized zeolite ZSM-45.

6. A process according to any of Claims 1 to 5 wherein said catalyst comprises zeolite ZSM-45 having its original cations replaced, at least in part, with a cation or a mixture of cations selected from hydrogen, ammonium, rare earth metals, and metals of Groups IA, IIA, IIIA, IVA, IB, IIB, IIIB, IVB, VIB and VIII of the Periodic Table of the Elements.

7. A process according to any of Claims 1 to 6 wherein the gaseous diluent is co-fed to the reaction zone with the organic reactants using a weight hourly space velocity for the diluent of from 0.003 to 20 $hr^{-1}$ at a molar ratio of gaseous diluent to organic reactants of from 0.05:1 to 40:1.

8. A process according to any of Claims 1 to 7 wherein the gaseous diluent is selected from hydrogen, water and combinations of hydrogen and water.

9. A process according to any of Claims 1 to 8 wherein a water diluent is co-fed to the reaction zone by utilizing an organic reactant feed comprising a methanol-water mixture.

10. A process according to any of Claims 1 to 9 wherein said conversion reaction conditions include a

temperature from 275°C to 600°C, a pressure from $10^4$ Pa to $3 \times 10^6$ Pa and a weight hourly space velocity of organic components of said feedstock from 0.1 $hr^{-1}$ to 30 $hr^{-1}$.

11. A process according to any of Claims 1 to 10 wherein said catalyst composition additionally comprises a binder for said ZSM-45 zeolite.

12. A process according to any of Claims 1 to 11 wherein the zeolite ZSM-45 component of said catalyst composition has a cyclohexane sorption capacity of 2.2 to 7.0 weight percent at 2666.44 Pa.

**Patentansprüche**

1. Verfahren zur Umwandlung einer organischen Reaktantenzufuhr, wobei die organischen Reaktanten Methanol, Methyläther oder Mischungen davon umfassen, zu einem $C_2$—$C_4$-Olefin enthaltenden Kohlenwasserstoffreaktionsprodukt, wobei bei dem Verfahren die organische Reaktantenzufuhr in einer Reaktionszone unter Methanol- oder Methyläther-Umwandlungsreaktionsbedingungen mit einer auf Zeolith basierenden Katalysatorzusammensetzung in Kontakt gebracht wird, dadurch gekennzeichnet, daß die Katalysatorzusammensetzung einen synthetischen, porösen, kristallinen Zeolith umfaßt, bezeichnet als ZSM-45, wobei der Zeolith bezogen auf eine wasserfreie Basis und auf die Molverhältnisse der Oxyde die Formel hat:

$$M_{2/n}O:Al_2O_3:xSiO_2,$$

worin x größer als 8 ist, M ein organisches oder metallisches Kation oder eine Kombination von organischen und metallischen Kationen ist und worin n die Wertigkeit jedes vorhandenen entsprechenden Kations ist, wobei der Zeolith eine Porenstruktur hat, um merkliche Mengen von Zyklohexan zu absorbieren und der Zeolith ein Pulver-Röntgendiagramm aufweist, das die folgenden kennzeichnenden Linien enthält:

| D-Netzebenen-Abstand (A) | Relative Intensität $I/I_0$ |
|---|---|
| 8,02±0,14 | stark-sehr stark |
| 5,07±0,09 | mittel-stark |
| 4,21±0,08 | mittel-stark |
| 4,01±0,07 | stark-sehr stark |
| 3,78±0,07 | mittel-stark |
| 3,11±0,06 | mittel-stark |
| 2,751±0,05 | mittel-stark |

und daß die organische Reaktantenzufuhr mit dem Katalysator in Gegenwart eines gasförmigen Verdünnungsmittels in Kontakt gebracht wird, das aus Wasser, Wasserstoff enthaltendem Gas, Stickstoff, Helium, Kohlenmonoxyde, Kohlendioxyd und Mischungen dieser Verdünnungsmittel ausgewählt ist.

2. Verfahren nach Anspruch 1, worin in der Katalysatorzusammensetzung die ZSM-45-Zeolithkomponente in der Form wie sie synthetisiert ist, eine Zusammensetzung bezogen auf eine wasserfreie Basis und auf die Mole der Oxyde pro Mol Aluminiumoxyd hat, die durch die Formel ausgedrückt wird:

$$(0,8—1,8)R_2O:(0,0—0,3)Na_2O:(0,0—0,5)K_2O:Al_2O_3:xSiO_2,$$

worin R ein einwertiges organisches Kation ist, das von einer 2-(hydroxyalkyl)trialkylammoniumverbindung abgeleitet wird, worin die Alkylgruppe aus ein oder zwei Kohlenstoffatomen besteht, und x größer als 8 ist, wobei der Zeolith ZSM-45 weiterhin durch ein Röntgendiagramm gekennzeichnet ist, das hauptsächlich die Werte aufweist, wie sie in Tabelle 1 der Beschreibung festgelegt sind.

3. Verfahren nach Anspruch 1 oder 2, wobei in der Formel des Zeolith x von größer als 8 bis 100 beträgt.

4. Verfahren nach Anspruch 2 oder 3, wobei in der Formel des Zeolith R ein organisches Kation ist, das von Cholinchlorid abgeleitet wird.

5. Verfahren nach einem der Ansprüche 2 bis 4, worin die Katalysatorzusammensetzung das Produkt der thermischen Behandlung des Zeolith ZSM-45, so wie er synthetisiert ist, umfaßt.

6. Verfahren nach einem der Ansprüche 1 bis 5, wobei der Katalysator Zeolith ZSM-45 umfaßt, dessen ursprüngliche Kationen zumindest teilweise durch ein Kation oder eine Mischung von Kationen ausgetauscht sind, die aus Wasserstoff, Ammonium, Metallen der Seltenen Erden und Metallen der Gruppen IA, IIA, IIIA, IVA, IB, IIB, IIIB, IVB, VIB und VIII des Periodensystems der Elemente ausgewählt sind.

7. Verfahren nach einem der Ansprüche 1 bis 6, worin das gasförmige Verdünnungsmittel gleichzeitig mit den organischen Reaktanten der Reaktionszone zugeführt wird, wobei eine stündliche Gewichts-Raum-Geschwindigkeit für das Verdünnungsmittel von 0,003 bis 20 $h^{-1}$ bei einem Molverhältnis des gasförmigen Verdünnungsmittels zu den organischen Reaktanten von 0,05:1 bis 40:1 verwendet wird.

8. Verfahren nach einem der Ansprüche 1 bis 7, wobei das gasförmige Verdünnungsmittel aus Wasserstoff, Wasser und Kombinationen von Wasserstoff und Wasser ausgewählt ist.

9. Verfahren nach einem der Ansprüche 1 bis 8, worin ein Wasser-Verdünnungsmittel gleichzeitig der Reaktionszone zugeführt wird, indem eine organische Reaktantenzufuhr verwendet wird, die eine Mischung von Methanol und Wasser umfaßt.

10. Verfahren nach einem der Ansprüche 1 bis 9, worin die Umwandlungsreaktionsbedigungen eine Temperatur von 275°C bis 600°C, einen Druck von $10^4$ bis $3\times10^6$ Pa und eine stündliche Gewichts-Raum-Geschwindigkeit der organischen Komponenten des Ausgangsmaterials von 0,1 bis 30 $h^{-1}$ umfassen.

11. Verfahren nach einem der Ansprüche 1 bis 10, worin die Katalysatorzusammensetzung zusätzliche ein Bindemittel für den Zeolith ZSM-45 umfaßt.

12. Verfahren nach einem der Ansprüche 1 bis 11, worin die Zeolithkomponente ZSM-45 der Katalysatorzusammensetzung eine Sorptionskapazität für Cyclohexan von 2,2 bis 7,0 Gew.-% bei 2666,44 Pa hat.

**Revendications**

1. Procédé pour convertir une charge organique destinée à réagir, comprenant les composés organiques méthanol, oxyde de méthyle ou leurs mélanges destinés à réagir, en un produit hydrocarboné de réaction contenant des oléfines en $C_2$—$C_4$, ledit procédé comprenant la mise en contact de ladite charge organique de réaction, dans une zone de réaction, dans des conditions de réaction de conversion du méthanol ou de l'oxyde de méthyle, avec une composition de catalyseur à base de zéolite, procédé caractérisé en ce que ladite composition de catalyseur comprend une zéolite cristalline poreuse synthétique applelée ZSM-45, ladite zéolite ayant pour formule, sur une base anhydre et en termes des rapports molaires des oxydes:

$$M_{2/n}O:Al_2O_3:xSiO_2$$

dans laquelle x est supérieur à 8, M désigne un cation organique ou de métal ou est une combinaison de cations organiques des deux métaux et n est la valence de chaque cation respectif présent, ladite zéolite ayant une structure des pores telle qu'elle absorbe de fortes quantités de cyclohexane et ladite zéolite ayant un spectre de diffraction des rayons X par poudre, contenant les raies importantes suivantes:

| D-distance entre plans (Å) | Intensité relative, $I/I_o$ |
|---|---|
| 8,02±0,14 | Forte-très forte |
| 5,07±0,09 | Moyenne-forte |
| 4,21±0,08 | Moyenne-forte |
| 4,01±0,07 | Forte-très forte |
| 3,78±0,07 | Moyenne-forte |
| 3,11±0,06 | Moyenne-forte |
| 2,751±0,05 | Moyenne-forte |

et en ce qu'on met l'alimentation organique destinée à réagir en contact avec ledit catalyseur, en présence d'un diluant gazeux choisi parmi l'eau, un gaz contenant de l'hydrogène, l'azote, l'hélium, le monoxyde de carbone, le dioxyde de carbone et des mélanges desdits diluants.

2. Procédé selon la revendication 1 dans lequel, dans la composition du catalyseur, le composant zéolite ZSM-45, sous sa forme telle qu'obtenue par synthèse, a sur base anhydre et en termes de moles d'oxydes par mole d'alumine, une composition exprimée par la formule:

$$(0,8—1,8)R_2O:(0,0—0,3)Na_2O:(0,0—0,5)K_2O:Al_2O_3:xSiO_2$$

dans laquelle R désigne un cation organique monovalent provenant d'un composé de 2-(hydroxyalkyl)-trialkylammonium dans lequel le groupe alkyle est composé d'un ou deux atomes de carbone et x est

supérieur à 8, ladite zéolite ZSM-45 étant encore caractérisée par un spectre de diffraction des rayons X présentant des valeurs sensiblement telles que celles présentées au tableau 1 de la description.

3. Procédé selon la revendication 1 ou la revendication 2, dans lequel, dans la formule de la zéolite, x va d'une valeur supérieure à 8 jusqu'à 100.

4. Procédé selon la revendication 2 ou la revendication 3, dans lequel, dans la formule de la zéolite, R est un cation organique provenant du chlorure de choline.

5. Procédé selon l'une quelconque des revendications 2 à 4, dans lequel ladite composition de catalyseur comprend le produit du traitement thermique de la zéolite ZSM-45 telle qu'obtenue par synthèse.

6. Procédé selon l'une quelconque des revendications 1 à 5, dans lequel ledit catalyseur comprend de la zéolite ZSM-45 ayant ses cations d'origine remplacés, au moins en partie, par un cation ou un mélange de cations choisi parmi l'hydrogène, un ion ammonium, les métaux des terres rares et les métaux des groupes IA, IIA, IIIA, IVA, IB, IIB, IIIB, IVB, VIB et VIII du Tableau Périodique des Eléments.

7. Procédé selon l'une quelconque des revendications 1 à 6, dans lequel le diluant gazeux est co-introduit dans la zone de réaction avec les corps organiques destinés à réagir, en utilisant pour le diluant une vitesse spatial horaire en poids de 0,003 à 20 $h^{-1}$ à un rapport molaire du diluant gazeux aux corps organiques destinés à réagir compris entre 0,05:1 et 40:1.

8. Procédé selon l'une quelconque des revendications 1 à 7, dans lequel le diluant gazeux est choisi parmi l'hydrogène, l'eau et les combinaisons d'hydrogène et d'eau.

9. Procédé selon l'une quelconque des revendications 1 à 8, dans lequel le diluant aqueux est co-introduit dans la zone de réaction par utilisation d'une alimentation organique en corps destinés à réagir comprenant un mélange méthanol-eau.

10. Procédé selon l'une quelconque des revendications 1 à 9, dans lequel lesdites conditions des réaction de conversion comprennent une température allant de 275°C à 600°C, une pression de $10^4$ Pa à $3\times10^6$ Pa et une vitesse spatiale horaire en poids de composants organiques de ladite alimentation allant de 0,1 $h^{-1}$ à 30 $h^{-1}$.

11. Procédé selon l'une quelconque des revendications 1 à 10, dans lequel ladite composition de catalyseur comprend en outre un liant pour ladite zéolite ZSM-45.

12. Procédé selon l'une quelconque des revendications 1 à 11, dans lequel le composant zéolite ZSM-45 de ladite composition de catalyseur a une capacité de sorption du cyclohexane de 2,2 à 7,0% en poids à 2 666,44 Pa.